# EUROPEAN PATENT APPLICATION

(11) **EP 1 470 795 A1**
(43) Date of publication of application: **27.10.2004**
(21) Application number: 04008460.0
(22) Date of filing: 07.04.2004
(51) Int. Cl.: A61F 2/06, A61L 31/16, A61L 31/12

(54) **Intravascular device for treatment of aneurysmal tissue**

(30) Priority: 25.04.2003 US 423192
(71) Applicant: Medtronic Vascular, Inc., Santa Rosa, California 95403 (US)
(72) Inventor: Brin, David S., Santa Rosa, California 95404 (US); Tseng, David, Santa Rosa, California 95405 (US)
(74) Representative: Zimmermann, Gerd Heinrich

(57) **Abstract**

While the present invention has been described with reference to specific embodiments, it should be understood by those skilled in the art that various changes may be made and equivalents may be substituted without departing from the true spirit and scope of the invention.

## Description

### FIELD OF THE INVENTION

The field of the invention is the treatment of vascular abnormalities, particularly the treatment of aortic aneurysms utilizing an intravascular treatment device.

### BACKGROUND OF THE INVENTION

Aortic aneurysms pose a significant medical problem for the general population. Aneurysms within the aorta presently affect between two and seven percent of the general population and the rate of incidence appears to be increasing. This form of atherosclerotic vascular disease (hardening of the arteries) is characterized by degeneration in the arterial wall in which the wall weakens and balloons outward by thinning. Until the affected artery is removed or bypassed, a patient with an aortic aneurysm must live with the threat of aortic aneurysm rupture and death.

One clinical approach for patients with an aortic aneurysm is aneurysm repair by endovascular grafting. Endovascular grafting involves the transluminal placement of a prosthetic arterial stent in the endoluminal position (within the lumen of the artery). To prevent rupture of the aneurysm, a stent of tubular construction is introduced into the aneurytic blood vessel, typically from a remote location through a catheter introduced into a major blood vessel in the leg.

When inserted and deployed in a vessel, a stent acts as a prosthesis to maintain the vessel open. The stent typically has the form of an open-ended tubular element and most frequently is configured to enable its expansion from an outside diameter which is sufficiently small to allow the stent to traverse the vessel to reach a site where it is to be deployed, to an outside diameter sufficiently large to engage the inner lining of the vessel for retention at the site.

The customary procedure is to install a stent at the aneurysmal site at the time of or shortly after an angioplasty or other procedure is performed. The stent is deployed by, e.g., radial expansion under outwardly-directed radial pressure exerted by active inflation of the balloon of a balloon catheter on which the stent is mounted. In other instances, passive spring characteristics of a pre-formed stent operate to deploy the device. The stent is thus expanded to engage the inner lining or inwardly-facing surface of the vessel wall with sufficient resilience to allow some contraction from full expansion size of the stent but also with sufficient stiffness so that the stent largely resists the natural recoil of the vessel wall--particularly at the ends of the stent where it encounters healthy vessel tissue.

Despite the effectiveness of endovascular grafting, once the aneurysmal site is bypassed, the aneurysm remains. The aortic tissue can continue to degenerate such that the aneurysm increases in size due to thinning of the medial connective tissue architecture of the aorta and loss of elastin. It is therefore an object of the present invention to achieve a greater success of aneurysm repair and healing.

### SUMMARY OF THE INVENTION

This object is solved by a device according to claim 1. Further advantages, aspects and improvements of the invention are disclosed in the description, the dependent claims and the drawings.

Embodiments according to the present invention addresses the problem of aneurysm repair, particularly the problem of continued breakdown of aortic aneurysmal tissue. A consequence of such continued breakdown is rupture of the aneurysm. Methods and apparatus for supporting or bolstering the aneurysmal site by implanting a stent, while supplying a pharmaceutical agent to aid in stabilizing and healing the aneurysmal tissue are provided.

Thus, in one embodiment according to the invention there is provided An intravascular treatment device, comprising a stent locatable adjacent to an aneurysmal site where the stent includes a time-release coating consisting essentially of a polymer and at least one therapeutic agent. In some embodiments of the invention, the polymer of the coating is biodegradable. In other embodiments, the coating of the polymer is not biodegradable. In other embodiments, the polymer is a pH- or temperature-sensitive polymer. Therapeutic agents that can be used according to the present invention include at least one therapeutic selected from a metalloproteinase inhibitor, cyclooxygenase-2 inhibitor, anti-adhesion molecule, tetracycline-related compound, beta blocker, NSAID, or an angiotensin converting enzyme inhibitor.

### BRIEF DESCRIPTION OF THE DRAWINGS

A more particular description of the invention, briefly summarized above, may be had by reference to the embodiments according to the invention described in the present specification and illustrated in the appended drawings. It is to be noted, however, that the specification and appended drawings illustrate only certain embodiments of this invention and are, therefore, not to be considered to be limiting of its scope. The invention may admit to equally effective embodiments as defined by the claims.

Figure 1 is a schematic view of a human aortal aneurysm.

Figure 2 is a partial sectional view of a descending aorta with a bifurcated stent graft placed therein.

### DETAILED DESCRIPTION

Reference will now be made in detail to exemplary embodiments according to the invention. While the invention will be described in conjunction with these embodiments, it is to be understood that the described embodiments are not intended to limit the invention solely and specifically to only those embodiments. On the contrary, the invention is intended to cover alternatives, modifications, and equivalents that may be included within the spirit and scope of the invention as defined by the attached claims.

Methods and apparatus for stabilizing and treating an aneurysmal site include implanting of a coated endovascular stent that delivers a bioactive amount of one or more therapeutic agents to the aneurysmal site. The stent is implanted in an individual in a typical manner, where the stent acts as a delivery vehicle to deliver one or more therapeutic agents to the aneurysmal site. Specifically, a compound comprised of a suitable polymer and the therapeutic agent or agents is used to coat the stent.

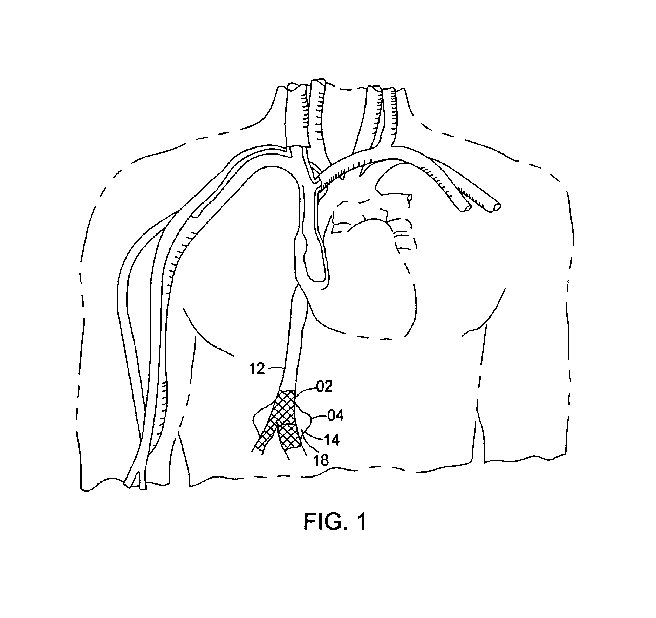

Referring initially to Figure 1, there is shown generally an aneurysmal blood vessel 10; in particular, there is an aneurysm of the aorta 12, such that the aorta or blood vessel wall 04 is enlarged at an aneurysmal site 14 and the diameter of the aorta 12 at the aneurysmal site 14 is on the order of over 100% to 300% of the diameter of a healthy aorta 12. The aneurysmal site 14 forms an aneurysmal bulge or sac 18. If left untreated, the aneurysmal sac 18 may continue to deteriorate, weaken, increase in size, and eventually tear or burst.

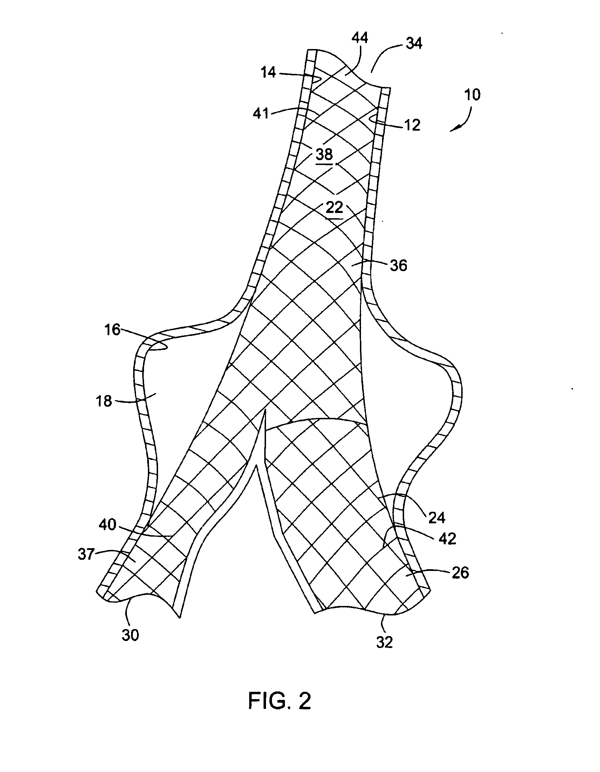

Figure 2 shows the transluminal placement of a prosthetic arterial stent graft 10, positioned in a blood vessel, in this embodiment, in, e.g., an abdominal aorta 12. The prosthetic arterial stent spans, within the aorta 12, an aneurysmal portion 14 of the aorta 12. The aneurysmal portion 14 is formed due to a bulging of the aorta wall 16, in a location where the strength and resiliency or the aorta wall 16 is weakened. As a result, an aneurysmal sac 18 is formed of distended vessel wall tissue. The stent graft 10 is positioned spanning the sac 18 providing both a secure passageway for blood flow through the aorta 12 and sealing of the aneurysmal portion 14 of the aorta 12 from additional blood flow from the aorta 12.

The placement of the stent graft 10 in the aorta 12 is a technique well known to those skilled in the art, and essentially includes opening a blood vessel in the leg or other remote location and inserting the stent graft 10 contained inside a catheter (not shown) into the blood vessel. The catheter/stent graft combination is tracked through the remote vessel until the stent graft 10 is deployed in a position that spans the aneurysmal portion 14 of aorta 12. The bifurcated stent graft 10 shown in Figure 2 has a pair of branched sections 20, 22 bifurcating from a trunk portion 24. This style of stent graft 10 is typically composed of two separate pieces, and is positioned in place first by inserting a catheter with the trunk portion 24 into place through an artery in one leg, providing a first branched section 20 to the aneurysmal location through the catheter and attaching it to the trunk portion at the aneurysmal site. Next, a second catheter with the second branched section 22 is inserted into place through an artery in the other leg of the patient, positioning the second branched section 22 adjacent to the trunk portion 24 and connecting it thereto. The procedure and attachment mechanisms for assembling the stent graft 10 in place in this configuration are well known in the art, and are disclosed in, e.g., Lombardi, et al., US Pat No. 6,203,568.

A coating compound provided is adapted to exhibit a combination of physical characteristics such as biocompatibility, and, preferably, biodegradability and bio-absorbability, while providing a delivery vehicle for release of one or more therapeutic agents that aid in the treatment of aneurysmal tissue. The coating compound used is biocompatible such that it results in no induction of inflammation or irritation when implanted, degraded or absorbed.

Thus, the coating according to the present invention may be either biodegradable or non-biodegradable. Representative examples of biodegradable compositions include albumin, collagen, gelatin, hyaluronic acid, starch, cellulose and cellulose derivatives (e.g., methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextran, polysaccharides, fibrinogen, poly(D,L-lactide), poly(D,L-lactide-co-glycolide), poly(glycolide), poly(hydroxybutyrate), poly(alkylcarbonate) and poly(orthoesters), polyesters, poly(hydroxyvaleric acid), polydioxanone, poly(ethylene terephthalate), poly(malic acid), poly(tartronic acid), polyanhydrides, polyphosphazenes, poly(amino acids) and their copolymers.

Representative examples of non-degradable polymers include poly(ethylene-vinyl acetate) ("EVA") copolymers, silicone rubber, acrylic polymers (polyacrylic acid, polymethylacrylic acid, polymethylmethacrylate, polyalkylcynoacrylate), polyethylene, polypropylene, polyamides (nylon 6,6), polyurethane, poly(ester urethanes), poly(ether urethanes), poly(ester-urea), polyethers (poly(ethylene oxide), poly(propylene oxide), pluronics and poly(tetramethylene glycol)), silicone rubbers and vinyl polymers (polyvinylpyrrolidone, poly(vinyl alcohol), poly(vinyl acetate phthalate). Polymers also may be developed which are either anionic (e.g., alginate, carboxymethyl cellulose and poly(acrylic acid), or cationic (e.g., chitosan, poly-L-lysine, polyethylenimine, and poly(allyl amine)).

Preferred polymeric coatings or carriers include poly(ethylene-vinyl acetate), polyurethanes, poly (D,L-lactic acid) oligomers and polymers, poly (L-lactic acid) oligomers and polymers, poly (glycolic acid), copolymers of lactic acid and glycolic acid, poly (caprolactone), poly (valerolactone), polyanhydrides, copolymers of poly (caprolactone) or poly (lactic acid) with a polyethylene glycol (e.g., MePEG), and blends, admixtures, or co-polymers of any of the above. Other preferred polymers include polysaccharides such as hyaluronic acid, chitosan and fucans, and copolymers of polysaccharides with degradable polymers.

Other polymers useful for these applications include carboxylic polymers, polyacetates, polyacrylamides, polycarbonates, polyethers, polyesters, polyethylenes, polyvinylbutyrals, polysilanes, polyureas, polyurethanes, polyoxides, polystyrenes, polysulfides, polysulfones, polysulfonides, polyvinylhalides, pyrrolidones, thermal-setting polymers, cross-linkable acrylic and methacrylic polymers, ethylene acrylic acid copolymers, styrene acrylic copolymers, vinyl acetate polymers and copolymers, vinyl acetal polymers and copolymers, epoxy, melamine, other amino resins, phenolic polymers, and copolymers thereof, water-insoluble cellulose ester polymers (including cellulose acetate propionate, cellulose acetate, cellulose acetate butyrate, cellulose nitrate, cellulose acetate phthalate, and mixtures thereof), polyvinylpyrrolidone, polyethylene glycols, polyethylene oxide, polyvinyl alcohol, polyethers, polysaccharides, hydrophilic polyurethane, polyhydroxyacrylate, dextran, xanthan, hydroxypropyl cellulose, methyl cellulose, and homopolymers and copolymers of N-vinylpyrrolidone, N-vinyllactam, N-vinyl butyrolactam, N-vinyl caprolactam, other vinyl compounds having polar pendant groups, acrylate and methacrylate having hydrophilic esterifying groups, hydroxyacrylate, and acrylic acid, and combinations thereof; cellulose esters and ethers, ethyl cellulose, hydroxyethyl cellulose, cellulose nitrate, cellulose acetate, cellulose acetate butyrate, cellulose acetate propionate, polyurethane, polyacrylate, natural and synthetic elastomers, rubber, acetal, nylon, polyester, styrene polybutadiene, acrylic resin, polyvinylidene chloride, polycarbonate, homopolymers and copolymers of vinyl compounds, polyvinylchloride, polyvinylchloride acetate. In general, see United States Pat. Nos. 6,514,515 to Williams; 6,506,410 to Park, et al.; 6,531,154 to Mathiowitz, et al.; 6,344,035 to Chudzik, et al.; 6,376,742 to Zdrahala, et al.; and Griffith, L.A., Ann. N.Y. Acad. of Sciences, 961:83-95 (2002); and Chaikof, et al, Ann. N.Y. Acad. of Sciences, 961:96-105 (2002).

Additionally, polymers as described herein can also be blended or copolymerized in various compositions as required.

The polymeric coatings as discussed can be fashioned in a variety of forms with desired release characteristics and/or with specific desired properties. For example, the polymeric coatings may be fashioned to release the therapeutic agent or agents upon exposure to a specific triggering event such as pH. Representative examples of pH-sensitive polymers include poly(acrylic acid) and its derivatives (including for example, homopolymers such as poly(aminocarboxylic acid); poly(acrylic acid); poly(methyl acrylic acid), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropyl methylcellulose acetate succinate; cellulose acetate trimellilate; and chitosan. Yet other pH sensitive polymers include any mixture of a pH sensitive polymer and a water-soluble polymer.

Likewise, polymeric carriers can be fashioned that are temperature sensitive. Representative examples of thermogelling polymers and their gelatin temperature include homopolymers such as poly(N-methyl-N-n-propylacrylamide)(19.8°C); poly(N-n-propylacrylamide)(21.5°C); poly(N-methyl-N-isopropylacrylamide)(22.3°C); poly(N-n-propylmethacrylamide(28.0°C); poly(N-isopropylacrylamide)(30.9°C); poly(N,n-diethylacrylamide)(32.0°C); poly(N-isopropylmethacrylamide)(44.0°C); poly(N-cyclopropylacrylamide)(45.5°C); poly(N-ethylmethyacrylamide)(50.0°C); poly(N-methyl-N-ethylacrylwnide)(56.0°C); poly(N-cyclopropylmethacrylamide)(59.0°C); poly(N-ethylacrylamide)(72.0°C). Moreover, thermogelling polymers may be made by preparing copolymers between (among) monomers of the above, or by combining such homopolymers with other water-soluble polymers such as acrylmonomers (e.g., acrylic acid and derivatives thereof such as methylacrylic acid, acrylate and derivatives thereof such as butyl methacrylate, acrylamide, and N-n-butyl acrylamide).

Other representative examples of thermogelling polymers include cellulose ether derivatives such as hydroxypropyl cellulose (41°C); methyl cellulose (55°C); hydroxypropylmethyl cellulose (66°C); and ethylhydroxyethyl cellulose, and Pluronics such as F-127 (10-15 °C); L-122 (19°C); L-92 (26°C); L-81 (20°C); and L-61 (24°C).

The polymer used may be obtained from various chemical companies known to those with skill in the art. However, because of the presence of unreacted monomers, low molecular weight oligomers, catalysts, and other impurities, it may be desirable (and, depending upon the materials used, may be necessary) to increase the purity of the polymer used. The purification process yields polymers of better-known, purer composition, and therefore increases both the predictability and performance of the mechanical characteristics of the coatings. The purification process will depend on the polymer or polymers chosed. Generally, in the purification process, the polymer is dissolved in a suitable solvent. Suitable solvents include (but are not limited to) methylene chloride, ethyl acetate, chloroform, and tetrahydrofuran. The polymer solution usually is then mixed with a second material that is miscible with the solvent, but in which the polymer is not soluble, so that the polymer (but not appreciable quantities of impurities or unreacted monomer) precipitates out of solution. For example, a methylene chloride solution of the polymer may be mixed with heptane, causing the polymer to fall out of solution. The solvent mixture then is removed from the copolymer precipitate using conventional techniques. For information regarding stents and coatings, see U.S. Pat. Nos. 6,387,121 to Alt; 6,451,373 to Hossainy, et al.; and 6,364,903 to Tseng, et al.

Generally, aneurysm results from the invasion of the cell wall by elastin-attacking proteins that occur naturally in the body, but for unknown reasons begin to congregate at certain blood vessel sites, attack the blood vessel structure and cause inflammation of the vessel. Generally, a plurality of enzymes, proteins and acids--all naturally occurring--interact through specific biochemical pathways to form elastin-attacking proteins or to promote the attachment or absorption of elastin-attacking proteins into the cell wall. The elastin-attacking proteins and the resulting breakdown of tissue and inflammation are leading causes of aneurysm formation.

For example, arachidonal acid, a naturally-occurring fatty acid found in blood vessel, forms the cytokine PGE2 in the presence of COX-2. PGE2, when taken up by macrophages, induces the expression of matrix metalloproteinase 9 (MMP-9), which is an elastin-attacking enzyme and a member of a family of metalloproteinases. Additionally, PGE2 is believed to contribute directly to inflammation in the blood vessel wall, furthering physiological stress in the blood vessel wall. Moreover, each of these elastin-attacking protein compounds is likely to be present on the surface of the blood vessel wall, as well as within the cellular matrix of the blood vessel. Thus, agents that work to reduce the attachment and integration of elastin-attacking and inflammatory compounds to the blood vessel also are of interest for use as a therapeutic.

The therapeutic agents described provide intervention in the aforementioned biochemical pathways and mechanisms, reduction in the level of the individual components responsible for aneurysmal growth, and elimination or limitation of the advance of the aneurysmal event. In particular, therapeutic agents are provided, alone or in combination, to address the inflammation- or elastin-attacking compounds, that cause the transition of a blood vessel from a healthy to an aneurysmal condition. The therapeutic agent or agents are released over time in the aneurysmal location, reducing the likelihood of further dilation and increasing the likelihood of successful repair of the aneurysm.

The therapeutic agents described are those useful in suppressing proteins known to occur in and contribute to aneurysmal sites, reducing inflammation at the aneurysmal site, and reducing the adherence of elastin-attacking proteins at the aneurysmal site. For example one class of materials, matrix metallproteinase (MMP) inhibitors, have been shown in some cases to reduce such elastin-attacking proteins directly, or in other cases indirectly by interfering with a precursor compound needed to synthesize the elastin-attacking protein. Another class of materials, NSAIDs, have demonstrated anti-inflammatory qualities that reduce inflammation at the aneurysmal site, as well as an ability to block MMP-9 formation. Further, yet another class of agents, attachment inhibitors, prevent or reduce the attachment or adherence of elastin-attacking proteins or inflammation-causing compounds onto the vessel wall at the aneurysmal site. Thus, these therapeutic agents and other such agents, alone or in combination, when provided at an aneurysmal site directly affect or undermine the underlying sequence of events leading to aneurysm formation and progression.

One class of agents useful in this application are those that block the formation of MMP-9 by interfering with naturally occurring body processes which yield MMP-9 as a byproduct. Cyclooxygenase-2 or "COX-2" is known to metabolize a fat in the body known as arachidonic acid or AA, a naturally occurring omega-6 fatty acid found in nearly all cell membranes in humans. Prostaglandin E2 (PGE2) is synthesized from the catalyzation of COX-2 and arachidonic acid and, when PGE2 is taken up by macrophages, it results in MMP-9 formation. Thus, if any of COX-2, PGE2, or AA are suppressed, then MMP-9 formation will be suppressed. Therefore, COX-2 inhibitors can be provided at the aneurysmal site. Such COX-2 inhibitors include Celecoxib, Rofecoxib and Parecoxib, all of which are available in pharmacological preparations. Additionally, COX-2 inhibition has been demonstrated from administration of herbs such as green tea, ginger, tumeric, chamomile, Chinese gold-thread, barberry, baikal skullcap, Japanese knotweed, rosemary, hops, feverfew, and oregano; and other agents such as piroxican, mefenamic acid, meloxican, nimesulide, diclofenac, MF-tricyclide, raldecoxide, nambumetone, naproxen, herbimycin-A, and etoicoxib, and it is specifically contemplated by the present invention that such additional COX-2 inhibiting materials may be formulated for use in an aneurysmal location.

In addition to inhibiting COX-2 formation, the generation of elastin-attacking proteins may be limited by interfering with the oxidation reaction between COX-2 and AA by reducing the capability of AA to oxidize. It is known that certain NSAIDs provide this function. For example, ketoralac tromethamine (Toradol) inhibits synthesis of progstaglandins including PGE2. In addition, other currently available NSAIDs, including indomethacin, ketorolac, ibuprofen and aspirin, among others, reduce inflammation at the aneurysmal site, limiting the ability of elastin attacking proteins such as MMP-9 to enter into the cellular matrix of the blood vessel and degrade elastin. Additionally, steroidal based anti-inflammatories, such as methylprednisolone or dexamethasone may be provided to reduce the inflammation at the aneuysmal site.

Despite the presence of inhibitors of COX-2 or of the oxidation reaction between COX-2 and AA; and/or despite the presence of an anti-inflammatory to reduce irritation and swelling of the blood vessel wall, MMP-9 may still be present in the blood vessel. Therefore, another class of therapeutic agents useful in this application are those that limit the ability of elastin-attacking proteins to adhere to the blood vessel wall, such as anti-adhesion molecules. Anti-adhesion molecules, such as anti-CD18 monoclonal antibody, limit the capability of leukocytes that may have taken up MMP-9 to attach to the blood vessel wall, thereby preventing MMP-9 from having the opportunity to enter into the blood vessel cellular matrix and attack the elastin.

In addition, other therapeutic agents contemplated to be used are tetracycline and related tetracycline-derivative compounds. In using a tetracycline compound as a bioactive agent in aneurysm treatment, the observed anti-aneurysmal effect appears to be unrelated to and independent of any antimicrobial activity such a compound might have. Accordingly, the tetracycline may be an antimicrobial tetracycline compound, or it may be a tetracycline analogue having little or no significant antimicrobial activity.

Preferred antimicrobial tetracycline compounds include, for example, tetracycline per se, as well as derivatives thereof. Preferred derivatives include, for example, doxycycline, aureomycin and chloromycin. If a tetracycline analogue having little or no antimicrobial activity is to be employed, it is preferred that the compound lack the dimethylamino group at position 4 of the ring structure. Such chemically-modified tetracyclines include, for example, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, and 6α-deoxy-5-hydroxy-4-dedimethylaminotetracycline. Also, tetracyclines modified at the 2-carbon position to produce a nitrile, e.g., tetracyclinonitrile, are useful as non-antibacterial, anti-metalloproteinase agents. Further examples of tetracyclines modified for reduced antimicrobial activity include 6-α-benzylthiomethylenetetracycline, the mono-N-alkylated amide of tetracycline, 6-fluoro-6-demethyltetracycline, and 11-α-chlorotetracycline.

Among the advantages is that the tetracycline compound is administered locally in an amount which has substantially no antibacterial activity, but which is effective for reducing pathology for inhibiting the undesirable consequences associated with aneurysms in blood vessels. Alternatively, as noted above, the tetracycline compound can have been modified chemically to reduce or eliminate its antimicrobial properties. The use of such modified tetracyclines is preferred in the present invention since they can be used at higher levels than antimicrobial tetracyclines, while avoiding certain disadvantages, such as the indiscriminate killing of beneficial microbes that often accompanies the use of antimicrobial or antibacterial amounts of such compounds.

Another class of therapeutic agent that finds utility in inhibiting the progression of or inducing the regression of a pre-existing aneurysm is beta blockers or beta adrenergic blocking agents. Beta blockers are bioactive agents that reduce the symptoms associated with hypertension, cardiac arrhythmias, angina pectoris, migraine headaches, and other disorders related to the sympathetic nervous system. Beta blockers also are often administered after heart attacks to stabilize the heartbeat. Within the sympathetic nervous system, beta-adrenergic receptors are located mainly in the heart, lungs, kidneys and blood vessels. Beta-blockers compete with the nerve-stimulated hormone epinephrine for these receptor sites and thus interfere with the action of epinephrine, lowering blood pressure and heart rate, stopping arrhythmias, and preventing migraine headaches. Because it is also epinephrine that prepares the body for "fight or flight", in stressful or fearful situations, beta-blockers are sometimes used as anti-anxiety drugs, especially for stage fright and the like. There are two main beta receptors, beta 1 and beta 2. Some beta blockers are selective, such that they selectively block beta 1 receptors. Beta 1 receptors are responsible for the heart rate and strength of the heartbeat. Nonselective beta blockers block both beta 1 and beta 2 receptors. Beta 2 receptors are responsible for the function of smooth muscle.

Beta blockers that may be used in the compounds and methods according to the present invention include acebutolol, atenolol, betaxolol, bisoprolol, carteolol, carvedilol, esmolol, labetolol, metoprolol, nadolol, penbutolol, pindolol, propranolol, and timolol, as well as other beta blockers known in the art.

In addition to therapeutic agents that inhibit elastases or reduce inflammation are agents that inhibit formation of angiotensin II, known as angiotensin converting enzyme (ACE) inhibitors. ACE inhibitors are known to alter vascular wall remodeling, and are used widely in the treatment of hypertension, congestive heart failure, and other cardiovascular disorders. In addition to ACE inhibitors' antihypertensive effects, these compounds are recognized as having influence on connective tissue remodeling after myocardial infarction or vascular wall injury.

ACE inhibitors prevent the generation of angiotensin-II, and many of the effects of angiotensin-II involve activation of cellular AT1 receptors; thus, specific AT1 receptor antagonists have also been developed for clinical application. ACE is an ectoenzyme and a glycoprotein with an apparent molecular weight of 170,000 Da. Human ACE contains 1277 amino acid residues and has two homologous domains, each with a catalytic site and a region for binding Zn⁺². ACE has a large amino-terminal extracellular domain and a 17-amino acid hydrophobic stretch that anchors the ectoenzyme to the cell membrane. Circulating ACE represents membrane ACE that has undergone proteolysis at the cell surface by a sectretase.

ACE is a rather nonspecific enzyme and cleaves dipeptide units from substrates with diverse amino acid sequences. Preferred substrates have only one free carboxyl group in the carboxyl-terminal amino acid, and proline must not be the penultimate amino acid. ACE is identical to kininase II, which inactivates bradkinin and other potent vasodilator peptides. Although slow conversion of angiotensin I to angiontensin II occurs in plasma, the very rapid metabolism that occurs in vivo is due largely to the activity of membrane-bound ACE present on the luminal aspect of the vascular system-- thus, the localized delivery of the ACE inhibitor contemplated by the present invention provides a distinct advantage over prior art systemic modes of administration.

Following the understanding of ACE, research focused on ACE inhibiting substances to treat hypertension. The essential effect of ACE inhibitors is to inhibit the conversion of relatively inactive angiotensin I to the active angiotensin II. Thus, ACE inhibitors attenuate or abolish responses to angiotensin I but not to angiotensin II. In this regard, ACE inhibitors are highly selective drugs. They do not interact directly with other components of the angiotensin system, and the principal pharmacological and clinical effects of ACE inhibitors seem to arise from suppression of synthesis of angiotensin II. Nevertheless, ACE is an enzyme with many substrates, and systemic administration of ACE inhibitors may not be optimal.

Many ACE inhibitors have been synthesized. Many ACE inhibitors are ester-containing prodrugs that are 100 to 1000 times less potent ACE inhibitors than the active metabolites but have an increased bioavailability for oral administraion than the active molecules.

Currently, twelve ACE inhibitors are approved for used in the United States. In general, ACE inhibitors differ with regard to three properties: (1) potency; (2) whether ACE inhibition is due primarily to the drug itself or to conversion of a prodrug to an active metabolite; and (3) pharmacokinetics (i.e., the extent of absorption, effect of food on absorption, plasma half-life, tissue distribution, and mechanisms of elimination). For example, with the notable exceptions of fosinopril and spirapril, which display balanced elimination by the liver and kidneys, ACE inhibitors are cleared predominantly by the kidneys. Therefore, impaired renal function inhibits significantly the plasma clearance of most ACE inhibitors, and dosages of such ACE inhibitors should be reduced in patients with renal impairment.

For systemic administration there is no compelling reason to favor one ACE inhibitor over another, since all ACE inhibitors effectively block the conversion of angiotensin I to angiontensin II and all have similar therapeutic indications, adverse-effect profiles and contraindications. However, there are preferred ACE inhibitors for use in the present invention. ACE inhibitors differ markedly in their activity and whether they are administered as a prodrug, and this difference leads to the preferred locally-delivered ACE inhibitors according to the present invention.

One preferred ACE inhibitor is captopril (Capoten). Captopril was the first ACE inhibitor to be marketed, and is a potent ACE inhibitor with a Ki of 1.7 nM. Captopril is the only ACE inhibitor approved for use in the United States that contains a sulfhydryl moiety. Given orally, captopril is rapidly absorbed and has a bioavailability of about 75%. Peak concentrations in plasma occur within an hour, and the drug is cleared rapidly with a half-life of approximately 2 hours. The oral dose of captopril ranges from 6.25 to 150 mg two to three times daily, with 6.25 mg three times daily and 25 mg twice daily being appropriate for the initiation of therapy for heart failure and hypertension, respectively.

Another preferred ACE inhibitor is lisinopril. Lisinopril (Prinivil, Zestril) is a lysine analog of enalaprilat (the active form of enalapril (described below)). Unlike enalapril, lisinopril itself is active. In vitro, lisinopril is a slightly more potent ACE inhibitor than is enalaprilat, and is slowly, variably, and incompletely (about 30%) absorbed after oral administration; peak concentrations in the plasma are achieved in about 7 hours. Lisinopril is cleared as the intact compound in the kidney, and its half-life in the plasma is about 12 hours. Lisinopril does not accumulate in the tissues. The oral dosage of lisinopril ranges from 5 to 40 mg daily (single or divided dosage), with 5 and 10 mg daily being appropriate for the initiation of therapy for heart failure and hypertension, respectively.

Enalapril (Vasotec) was the second ACE inhibitor approved in the United States. However, because enalapril is a prodrug that is not highly active and must be hydrolyzed by esterases in the liver to produce enalaprilat, the active form, enalapril is not a preferred ACE inhibitor of the present invention. Similarly, fosinopril (Monopril), benazepril (Lotensin), fosinopril (Monopril), trandolapril (Mavik) (quinapril (Accupril), ramipril (Altace), moexipirl (Univasc) and perindopril (Aceon) are all prodrugs that require cleavage by hepatic esterases to transform them into active, ACE-inhibiting forms, and are not preferred ACE inhibitors. However, the active forms of these compounds (i.e., the compounds that result from the prodrugs being converted by hepatic esterases)--namely, enalaprilat (Vasotec injection), fosinoprilat, benazeprilat, trandolaprilat, quinaprilat, ramiprilat, moexiprilat, and perindoprilat―are suitable for use, and because of the localized drug delivery, the bioavailability issues that affect the oral administration of the active forms of these agents are moot.

The maximal dosage of the therapeutic to be administered is the highest dosage that effectively inhibits elastolytic, inflammatory or other aneurysmal activity, but does not cause undesirable or intolerable side effects. For example, a tetracycline compound can be administered in an amount of from about 0.1 mg/kg/day to about 30 mg/kg/day, and preferably from about 1 mg/kg/day to about 18 mglkg/day. For the purpose of embodiments according to the present invention, side effects include clinically significant antimicrobial or antibacterial activity, as well as toxic effects. For example, a dose in excess of about 50 mg/kg/day would likely produce side effects in most mammals, including humans. The dosage of the therapeutic agent or agents used will vary depending on properties of the coating, including its time-release properties, whether the coating is itself biodegradable, and other properties. Also, the dosage of the therapeutic agent or agents used will vary depending on the potency, pathways of metabolism, extent of absorption, half-life, and mechanisms of elimination of the therapeutic agent itself. In any event, the practitioner is guided by skill and knowledge in the field, and embodiments according to the present invention include without limitation dosages that are effective to achieve the described phenomena.

The therapeutic agent or agents may be linked by occlusion in the matrices of the polymer coating, bound by covalent linkages, or encapsulated in microcapsules. Within certain embodiments, the therapeutic agent or agents are provided in noncapsular formulations such as microspheres (ranging from nanometers to micrometers in size), pastes, threads of various size, films and sprays.

Within certain aspects, the coating is formulated to deliver the therapeutic agent or agents over a period of several hours, days, or, months. For example, "quick release" or "burst" coatings are provided that release greater than 10%, 20%, or 25% (w/v) of the therapeutic agent or agents over a period of 7 to 10 days after deployment. Within other embodiments, "slow release" therapeutic agent or agents are provided that release less than 1% (w/v) of a therapeutic agent over a period of 7 to 10 days. Further, the therapeutic agent or agents of the present invention should preferably be stable for several months and capable of being produced and maintained under sterile conditions.

Within certain aspects, therapeutic coatings may be fashioned in any size ranging from 50 nm to 500 µm, depending upon the particular use. Alternatively, such compositions may also be readily applied as a "spray", which solidifies into a film or coating. Such sprays may be prepared from microspheres of a wide array of sizes, including for example, from 0.1 µm to 3 µm, from 10 µm to 30 µm, and from 30 µm to 100 µm.

The therapeutic agent or agents of the present invention also may be prepared in a variety of "paste" or gel forms. For example, within one embodiment of the invention, therapeutic coatings are provided which are liquid at one temperature (e.g., temperature greater than 37°C, such as 40°C, 45°C, 50°C, 55°C or 60°C), and solid or semi-solid at another temperature (e.g., ambient body temperature, or any temperature lower than 37°C). Such "thermopastes" readily may be made utilizing a variety of techniques. Other pastes may be applied as a liquid, which solidify in vivo due to dissolution of a water-soluble component of the paste and precipitation of encapsulated drug into the aqueous body environment.

Within yet other aspects, the therapeutic compositions of the present invention may be formed as a film. Preferably, such films are generally less than 5, 4, 3, 2, or 1 mm thick, more preferably less than 0.75 mm, 0.5 mm, 0.25 mm, or, 0.10 mm thick. Films can also be generated of thicknesses less than 50 µm, 25 µm or 10 µm. Such films are preferably flexible with a good tensile strength (e.g., greater than 50, preferably greater than 100, and more preferably greater than 150 or 200 N/cm²), have good adhesive properties (i.e., adhere to moist or wet surfaces), and have controlled permeability.

Within certain embodiments, the therapeutic compositions may also comprise additional ingredients such as surfactants (e.g., pluronics, such as F-127, L-122, L-101, L-92, L-81, and L-61).

In one embodiment, the coating is coated with a physical barrier. Such barriers can include inert biodegradable materials such as gelatin, PLGA/MePEG film, PLA, or polyethylene glycol among others. In the case of PLGA/MePEG, once the PLGA/MePEG becomes exposed to blood, the MePEG will dissolve out of the PLGA, leaving channels through the PLGA to underlying layer of biologically active substance (e.g., poly-1-lysine, fibronectin, or chitosan), which then can initiate its biological activity.

Protection of the therapeutic coating also can be utilized by coating the surface with an inert molecule that prevents access to the active site through steric hindrance, or by coating the surface with an inactive form of the biologically active substance, which is later activated. For example, the coating further can be coated readily with an enzyme, which causes either release of the therapeutic agent or agents or activates the therapeutic agent or agents. Indeed, alternating layers of the therapeutic coating with a protective coating may enhance the time-release properties of the coating overall.

Another example of a suitable second coating is heparin, which can be coated on top of therapeutic agent-containing coating. The presence of heparin delays coagulation. As the heparin or other anticoagulant dissolves away, the anticoagulant activity would stop, and the newly exposed therapeutic agent-containing coating could initiate its intended action.

In another strategy, the stent graft can be coated with an inactive form of the therapeutic agent or agents, which is then activated once the stent graft is deployed. Such activation could be achieved by injecting another material into the aneurysm sac after the stent graft is deployed. In this iteration, the graft material could be coated with an inactive form of the therapeutic agent or agents, applied in the usual manner. Prior to the deployment of the aortic segment of the device, a catheter would be placed within the aneurysm sac via the opposite iliac artery, via an upper limb vessel such as a brachial artery, or via the same vessel as the aortic segment is inserted through so that once the stent graft is deployed, this catheter will be inside the aneurysm sac, but outside the stent graft. The stent graft would then be deployed in the usual manner. Once the stent graft is fully deployed, excluding the aneurysm, the activating substance is injected into the aneurysm sac around the outside of the stent graft.

Stents are designed to be deployed and expanded in different ways. A stent can be designed to self expand upon release from its delivery system, or it may require application of a radial force through the delivery system to expand the stent to the desired diameter. Self expanding stents are typically made of metal and are woven, wound like a spring, or otherwise configured to be compressible. Synthetic polymer stents of this type are also known in the art. Self-expanding stents are compressed prior to insertion into the delivery device and released by the practitioner when correctly positioned within the stricture site. After release, the stent self expands to a predetermined diameter and is held in place by the expansion force or other physical features of the device.

Stents which require mechanical expansion by the surgeon are commonly deployed by a balloon-type catheter. Once positioned within the stricture, the stent is expanded *in situ* to a size sufficient to fill the lumen and prevent restenosis. Various designs and other means of expansion have also been developed.

Alternatively, to deploy the stent in one embodiment of according to the present invention, the stent may be in its unexpanded state along the periphery of a balloon portion of a balloon catheter, and inserted into the body lumen percutaneously where it travels through the body lumen to the desired site for deployment. Once at the desired site, the stent is heated for the requisite period of time until its temperature is above the glass-transition temperature of the copolymer, and the balloon then is expanded so that the stent expands to the required size. The heat source then is removed, and the stent cooled by convection and conduction until its temperature is reduced below the glass-transition temperature. (Alternatively, but less desirably, a cooling medium may optionally be introduced through the catheter to cool the stent below the glass-transition temperature.) The stent may also be expanded slightly during this cooling process to fix the circumferential alignment of the polymer chains and prevent strain recovery (shrinkage) of the expanded stent. One such heating technique is described generally in Lee, U.S. Pat. No. 5,292,321 (which is incorporated by reference herein). Another heating technique that may be used in the invention is described generally in Rappaport, U.S. Pat. No. 5,470,352, as a balloon catheter including a microwave antenna. These embodiments reveal that the invention is not limited to a single method of deployment.

While the present invention has been described with reference to specific embodiments, it should be understood by those skilled in the art that various changes may be made and equivalents may be substituted without departing from the true spirit and scope of the invention. In addition, many modifications may be made to adapt a particular situation, material, or process to the objective, spirit and scope of the present invention. All such modifications are intended to be within the scope of the invention.

All references cited herein are to aid in the understanding of the invention, and are incorporated in their entireties for all purposes.

## Claims

1. An intravascular treatment device, comprising:
a stent locatable adjacent to an aneurysmal site; wherein the stent includes a time release coating consisting essentially of a polymer and at least one therapeutic agent.

2. The treatment device of claim 1, wherein the polymer is biodegradable.

3. The treatment device of claim 2, wherein the polymer is collagen, gelatin, hyaluronic acid, starch, cellulose, cellulose derivatives, casein, dextran, polysaccharide, fibrinogen, poly(D,L-lactide), poly(D,L-lactide-co-glycolide), poly(glycolide), poly(hydroxybutyrate), poly(alkylcarbonate), poly(orthoesters), polyester, poly(hydroxyvaleric acid), polydioxanone, poly(ethylene terephthalate), poly(malic acid), poly(tartronic acid), polyanhydride, polyphosphazene, poly(amino acids) or their copolymers.

4. The treatment device of claim 1, wherein the polymer is not biodegradable.

5. The treatment device of claim 4, wherein the polymer is poly(ethylene-vinyl acetate), silicone rubber, acrylic polymer, polyethylene, polypropylene, polyamide, nylon 6,6, polyurethane, poly(ester urethane), poly(ether urethanes, poly(ester-urea), polyethers (poly(ethylene oxide), poly(propylene oxide), pluronics, poly(tetramethylene glycol)), silicone rubber, or vinyl polymer.

6. The treatment device of claim 1, wherein the polymer is poly(ethylene-vinyl acetate), polyurethane, poly (D,L-lactic acid) oligomers or polymers, poly (L-lactic acid) oligomers or polymers, poly (glycolic acid), copolymers of lactic acid and glycolic acid, poly (caprolactone), poly (valerolactone), polyanhydride, copolymers of poly (caprolactone) or poly (lactic acid) with a polyethylene glycol, or blends, admixtures, or co-polymers of any of the above.

7. The treatment device of claim 1, wherein the polymer is hyaluronic acid, chitosan or fucans.

8. The treatment device of claim 1, wherein the polymer is a pH-sensitive polymer.

9. The treatment device of claim 8, wherein the pH-sensitive polymer is poly(acrylic acid) or its derivatives; poly(acrylic acid); poly(methyl acrylic acid), copolymers of poly(acrylic acid) and acrylmonomers; cellulose acetate phthalate; hydroxypropylmethylcellulose phthalate; hydroxypropyl methylcellulose acetate succinate; cellulose acetate trimellilate; or chitosan.

10. The treatment device of claim 1, wherein the polymer is a temperature-sensitive polymer.

11. The treatment device of claim 10, wherein the temperature-sensitive polymer is poly(N-methyl-N-n-propylacrylamide; poly(N-n-propylacrylamide); poly(N-methyl-N-isopropylacrylamide); poly(N-n-propylmethacrylamide; poly(N-isopropylacrylamide); poly(N,n-diethylacrylamide); poly(N-isopropylmethacrylamide); poly(N-cyclopropylacrylamide); poly(N-ethylmethyacrylamide); poly(N-methyl-N-ethylacrylamide); poly(N-cyclopropylmethacrylamide); poly(N-ethylacrylamide); hydroxypropyl cellulose; methyl cellulose; hydroxypropylmethyl cellulose; and ethylhydroxyethyl cellulose, or pluronics F-127; L-122; L-92; L-81; or L-61 or copolymers thereof.

12. The treatment device of claim 1, wherein the therapeutic agent is at least one of a metalloproteinase inhibitor, cyclooxygenase-2 inhibitor, anti-adhesion molecule, tetracycline-related compound, beta blocker, NSAID, or an angiotensin converting enzyme inhibitor.

13. The treatment device of claim 12, wherein the cyclooxygenase-2 inhibitor is Celecoxib, Rofecoxib, Parecoxib, green tea, ginger, tumeric, chamomile, Chinese gold-thread, barberry, baikal skullcap, Japanese knotweed, rosemary, hops, feverfew, oregano, piroxican, mefenamic acid, meloxican, nimesulide, diclofenac, MF-tricyclide, raldecoxide, nambumetone, naproxen, herbimycin-A, or etoicoxib.

14. The treatment device of claim 12 or 13, wherein the anti-adhesion molecule is anti-CD 18 monoclonal antibody.

15. The treatment device of claim 12 to 14, wherein the tetracycline-related compound is doxycycline, aureomycin, chloromycin, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, 6α-deoxy-5-hydroxy-4-dedimethylaminotetracycline, tetracyclinonitrile, 6-α-benzylthiomethylenetetracycline, 6-fluoro-6-demethyltetracycline, or 11-α-chlorotetracycline.

16. The treatment device of claim 12 to 15, wherein the beta blocker is acebutolol, atenolol, betaxolol, bisoprolol, carteolol, carvedilol, esmolol, labetolol, metoprolol, nadolol, penbutolol, pindolol, propranolol, or timolol.

17. The treatment device of claim 12 to 16, wherein the NSAID is indomethacin, ketorolac, ibuprofen or aspirin.

18. The treatment device of claim 12 to 17, wherein the angiotensin converting enzyme inhibitor is captopril, lisinopril, enalaprilat, fosinoprilat, benazeprilat, trandolaprilat, quinaprilat, ramiprilat, moexiprilat, or perindoprilat.

19. The treatment device according to any one of the preceding claims, wherein the therapeutic agent is linked by an occlusion in the coating polymer.

20. The treatment device according to any one of the preceding claims, wherein the therapeutic agent is bound by covalent linkages to the polymer.

21. The treatment device according to any one of the preceding claims, wherein the therapeutic agent is contained in a microsphere associated with the polymer.

22. The treatment device of claim 21, wherein in microsphere is about 50 nm to 500 µm in size.

23. The treatment device according to any one of the preceding claims, wherein the coating is applied as a paste, thread, film or spray.

24. The treatment device of claim 23, wherein the spray is prepared from microspheres of about 0.1 µm to about 100 µm in size.

25. The treatment device of claim 23 or 24, wherein the film is from 10 µm to 5 mm thick.

26. The treatment device according to any one of the preceding claims, further comprising a second coating deposed over the time release coating.

27. The treatment device of claim 26, wherein there are at least two time release coatings, wherein each time release coating is separated by a second coating.

28. The treatment device according to any one of the preceding claims, wherein the time release coating releases from about 1 % to about 25% of the therapeutic agent in the first 10 days.
